(19) Europäisches Patentamt
European Patent Office
Office européen des brevets

(11) Numéro de publication: **0 200 605**
**A1**

(12) **DEMANDE DE BREVET EUROPEEN**

(21) Numéro de dépôt: **86400708.3**

(22) Date de dépôt: **02.04.86**

(51) Int. Cl.[4]: **C 07 D 405/12**
**A 61 K 31/44**

(30) Priorité: **11.04.85 FR 8505424**

(43) Date de publication de la demande:
**05.11.86 Bulletin 86/45**

(84) Etats contractants désignés:
AT BE CH DE FR GB IT LI LU NL SE

(71) Demandeur: **SYNTHELABO**
**58, rue de la Glacière**
**F-75621 Paris Cedex 13(FR)**

(72) Inventeur: **Manoury, Philippe**
**L'Orée de Verrières 38, Avenue des Vaupépins**
**F-91370 Verrieres le Buisson(FR)**

(72) Inventeur: **Obitz, Daniel**
**91, rue Boucicaut**
**F-92260 Fontenay aux Roses(FR)**

(72) Inventeur: **Peynot, Michel**
**2, Rue des Marguerites**
**F-94240 L'Hay les Roses(FR)**

(74) Mandataire: **Thouret-Lemaitre, Elisabeth et al,**
**Service Brevets - SYNTHELABO 58, rue de la Glacière**
**F-75621 Paris Cedex 13(FR)**

(54) **Dérivés de nitrofuranne, leur préparation et leur application en thérapeutique.**

(57) Dérivés du nitrofuranne, répondant à la formule (I)

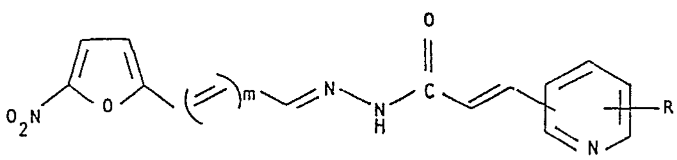

(I)

dans laquelle m est 0 ou 1 et
R est H ou $(C_{1-4})$alcoxy.
Application en thérapeutique.

EP 0 200 605 A1

La présente invention a pour objet des dérivés du nitrofuranne, leur préparation et leur application en thérapeutique.

Les composés de l'invention répondent à la formule générale (I) donnée en annexe 1
dans laquelle m est 0 ou 1 et
R est H ou $(C_{1-4})$alcoxy.

Selon l'invention, on peut préparer les composés (I) selon le schéma réactionnel de l'annexe 1 par condensation entre l'hydrazide correspondant (II) et le nitro-5 furannecarboxaldéhyde-2 (ou son vinylogue) dans un solvant alcoolique (comme le méthanol, l'éthanol ou le méthoxy-2 éthanol) maintenu entre 20°C et sa température de reflux. Les dérivés obtenus cristallisent spontanément dans le milieu réactionnel.

Le (nitro-5 furannyl-2)-3 propène-2 al est préparé par une réaction d'aldolisation entre le nitro-5 furannecarboxaldéhyde-2 et l'acétaldéhyde suivie d'une purification par chromatographie sur colonne de silice.

Les hydrazides peuvent être préparés par réaction entre les acides et l'hydrazine sèche en présence d'agents de condensation (comme par exemple le dicyclohexylcarbodiimide, l'E.E.D.Q. etc) ou bien par réaction entre l'hydrazine et un dérivé activé de l'acide (comme par exemple un anhydride mixte, un imidazolide, etc.).

Les acides éthyléniques sont préparés à partir des aldéhydes correspondants par condensation avec des dérivés d'esters ou d'acides à méthylène activé (comme par exemple dans les réactions de Doebner, de Perkin, de Wittig-Horner, etc.)

Les exemples suivants illustrent l'invention.

Les analyses et les spectres IR et RMN confirment la structure des composés.

Exemple 1. [(Nitro-5 furannyl-2)méthylène]hydrazide de l'acide (méthoxy-6 pyridinyl-3)-3 propène-2 oïque.

1.1. Acide (méthoxy-6 pyridinyl-3)-3 propène-2 oïque.

On chauffe à 100°C, pendant 1 h, un mélange de 11,5 g (0,084 mole) de méthoxy-6 pyridinecarboxaldéhyde-3 (décrit par I. Kompis et coll ; Europ. J. Med. chem., Chimie Therap (1977) 12(6) p 531-6) avec 8,75 g (0,084 mole) d'acide malonique, 6,65 g (0,084 mole) de pyridine et une goutte de pipéridine.

Puis on ajoute 100 ml d'eau et ajuste le pH à 4 avec de l'acide acétique. essore le solide, le lave à l'eau et le sèche. On obtient le composé qui fond à 190-1°C.

On prépare de la même manière les acides éthyléniques suivants :

- acide (pyridinyl-4)-3 propène-2 oïque : F = 296-8°C
- acide (pyridinyl-2)-3 propène-2 oïque : F = 202-3°C
- acide (méthoxy-5-pyridinyl-2)-3 propène-2 oïque : F = 217-8°C.

1.2. Hydrazide de l'acide (méthoxy-6 pyridinyl-3)-3 propène-2 oïque.

A une solution de 4,4 g (24,55 mmoles) de l'acide (méthoxy-6 pyridinyl-3)-3 propène-2 oïque dans 200 ml de chloroforme, on ajoute 2,5 g (24.6 mmoles) de triéthylamine, puis refroidit la solution résultante entre 0 et 5°C. On ajoute alors. goutte à goutte, une solution de 2,7 g (24,6 mmoles) de chloroformiate d'éthyle dans 20 ml de chloroforme, puis laisse agiter 1 h à cette température.

On y ajoute alors en une fois 3,2 g (0,1 mole) d'hydrazine sèche et laisse reposer 4 h à la température ambiante. On évapore alors le solvant jusqu'à siccité puis reprend le résidu par 50 ml d'eau bouillante, agite quelques instants en présence de noir animal et filtre à chaud cette

suspension. Par refroidissement entre 5 et 10°C, un solide cristallise que l'on essore, lave avec peu d'eau glacée puis sèche.
On obtient le composé qui fond à 173-4°C.

On prépare de la même manière les hydrazides suivants :
- hydrazide de l'acide (pyridinyl-3)-3 propène-2 oïque : F = 149-50°C
- hydrazide de l'acide (pyridinyl-4)-3 propène-2 oïque : F = 105-7°C
- hydrazide de l'acide (pyridinyl-2)-3 propène-2 oïque : F = 98-9°C
- hydrazide de l'acide (méthoxy-5 pyridinyl-2)-3 propène-2 oïque : F = 144-5°C.

1.3. <u>[(Nitro-5 furannyl-2)méthylène] hydrazide de l'acide(méthoxy-6 pyridinyl-3)-3 propène-2 oïque.</u>

A une solution, chauffée à 50-60°C, de 0,9 g (4,66 mmoles) de l'hydrazide de l'acide (méthoxy-6 pyridinyl-3)-3 propène-2 oïque dans 50 ml de méthanol, on ajoute 0,66 g (4,7 mmoles) de nitro-5 furannecarboxaldéhyde-2.

Après 1 h d'agitation pendant laquelle la température du milieu retombe à la température ambiante, le produit cristallisé qui a précipité est essoré, lavé à l'éther puis séché sous pression réduite à 100°C durant 8 h.
Le composé obtenu fond à 239-240°C.

<u>Exemple 2</u>. [(Nitro-5 furannyl-2)propène-2 ylidène-1] hydrazide de l'acide(méthoxy-6 pyridinyl-3)-3 propène-2 oïque.

A une solution tiède de 0.85 g (4,4 mmole) de l'hydrazide de l'acide (méthoxy-6 pyridinyl-3)-3 propène-2 oïque dans 50 ml de méthanol, on ajoute 0,75 g (4,5 mmole) de (nitro-5 furannyl-2)-3 propène-2 al. Au sein de la solution homogène résultante cristallise lentement un solide. Après agitation plusieurs heures on essore ces cristaux, les lave au

méthanol et à l'éther et les sèche sous pression réduite à 100° durant 8h.

Le composé obtenu fond à 232-3°C.

Les composés préparés à titre d'exemples sont représentés dans le tableau suivant.

Tableau

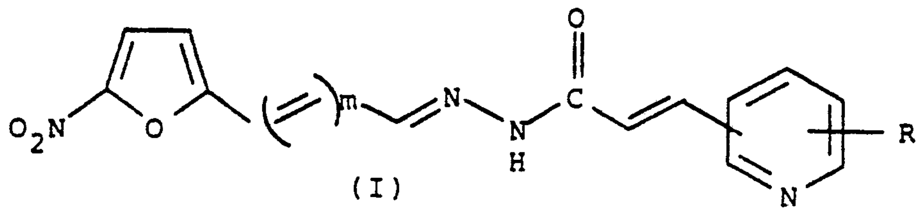

(I)

| Composé | m | position chaîne sur noyau pyridyle | R | F(°C) |
|---|---|---|---|---|
| 1 | 1 | 3 | H | 214-215 |
| 2 | 0 | 4 | H | 248-250 |
| 3 | 1 | 4 | H | 234-235 |
| 4 | 0 | 2 | H | 223-225 |
| 5 | 1 | 2 | H | 226-228 |
| 6 | 0 | 3 | $6-O-CH_3$ | 239-240 |
| 7 | 1 | 3 | $6-O-CH_3$ | 232-233 |
| 8 | 1 | 2 | $5-O-CH_3$ | 229-230 |

Les composés de l'invention ont été soumis à des essais pharmacologiques dans le domaine antibactérien, antiparasitaire et antifongique.

Les composés de l'invention présentent une activité inhibitrice "in vitro" et "in vivo" vis-à-vis d'un grand nombre de souches comprenant notamment : Staphylococcus aureus, Escherichia Coli, Mycobacterium ranae,Pseudomonas aeruginosa, Proteus Vulgaris, Vibrio Cholerae, Klebsiella pneumoniae, Trichomonas, Salmonella, Shighella Flexneri, Candida Albicans, qui permettent d'envisager leur utilisation dans diverses infections bactériennes et parasitaires, notamment au niveau intestinal.

La concentration minimale inhibitrice "in vitro" déterminée après mise en solution des composés dans le diméthylformamide (0,1 %) varie suivant les souches de 0,05µg/ml à 20µg/ml.

Les études réalisées "in vivo" sur l'infection expérimentale chez la souris démontrent l'activité orale des composés puisque ceux-ci inhibent la mortalité induite par plusieurs souches de bactéries et provoquent une stérilisation complète du tube digestif de la souris.

Les composés présentent une très faible toxicité qui est en général très supérieure à 1 g/kg.

Les composés de l'invention peuvent être utilisés en clinique chez l'homme à des doses de 20 mg à 1 g/jour, la posologie unitaire étant comprise entre 5 et 200 mg ; les composés peuvent être utilisés chez l'animal à des doses de 1 à 20 mg/kg/jour.

Les composés peuvent être présentés sous toute forme appropriée pour l'administration par voie orale, rectale ou parentérale, par exemple sous la forme de capsules, comprimés, granulés, gélules ou solutés liquides, sirops ou suspensions buvables, et contenir les excipients appropriés.

Les composés de l'invention peuvent être utilisés chez l'animal et l'homme comme antibactériens, antiseptiques intestinaux,antifongiques et/ou antiprotozoaires. Au niveau intestinal chez l'homme, ils pourront être employés pour soigner les colopathies fonctionnelles infectieuses, les diarhées d'origine alimentaire ou non, les entérites, les enterocolites, les dysenteries bacillaires.

Les composés de l'invention peuvent être également utilisés pour la protection et la conservation des aliments et pour favoriser la croissance du bétail, en luttant contre les infections bactériennes et parasitaires.

Annexe 1

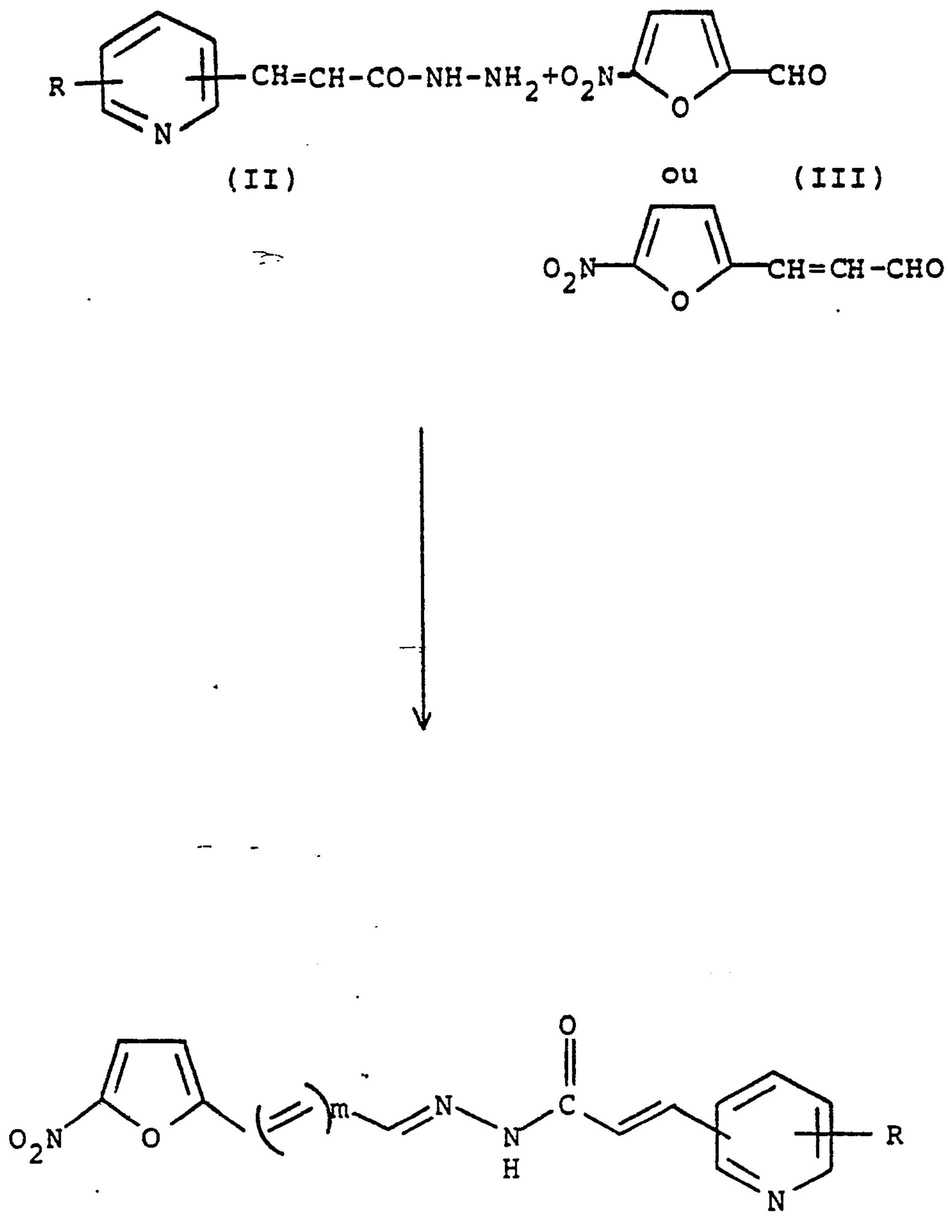

(II)

ou

(III)

(I)

Revendications pour les états contractants : BE, CH, DE, FR, GB, IT, LI, NL, SE.

1. Dérivés du nitrofuranne, répondant à la formule (I)

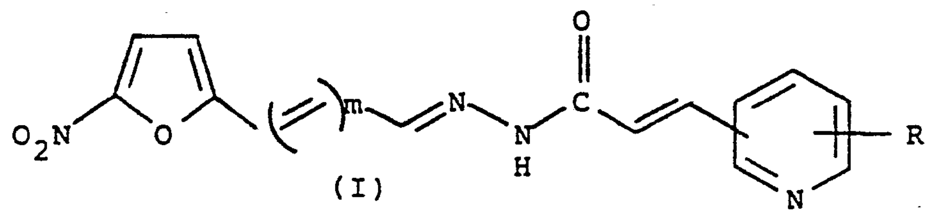

(I)

dans laquelle m est 0 ou 1 et R est H ou $(C_{1-4})$alcoxy.

2. Procédé de préparation des composés selon la revendication 1, procédé caractérisé en ce que l'on condense un hydrazide de formule (II)

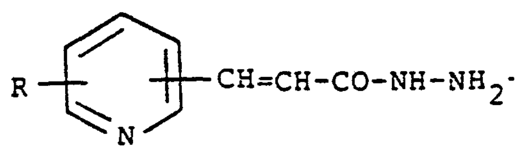

(II)

avec le nitro-5 furannecarboxaldéhyde-2 ou le (nitro-5 furyl-2)-3 propène-2 al, dans un solvant alcoolique, à une température allant de 20°C à la température de reflux du solvant.

3. Médicament caractérisé en ce qu'il contient un composé tel que spécifié dans la revendication 1.

4. Composition pharmaceutique caractérisée en ce qu'elle contient un composé tel que spécifié dans la revendication 1 en association avec tout excipient approprié.

Revendication pour l'état contractant : AT.

Procédé de préparation de composés répondant à la formule (I)

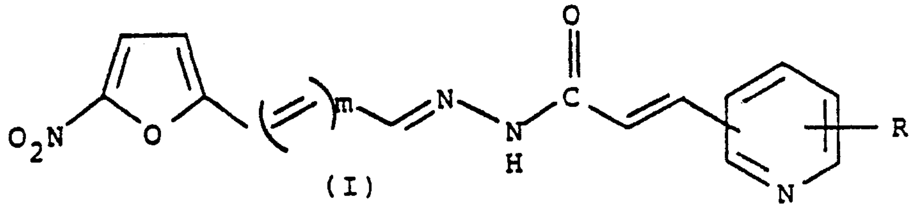

(I)

dans laquelle m est 0 ou 1 et

R est H ou $(C_{1-4})$alcoxy,

procédé caractérisé en ce que l'on condense un hydrazide de formule (II)

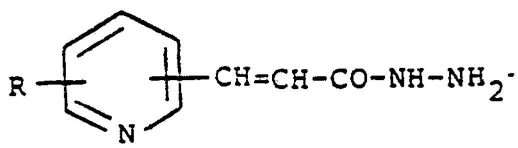

(II)

avec le nitro-5 furannecarboxaldéhyde-2 ou le (nitro-5 furyl-2)-3 propène-2 al, dans un solvant alcoolique, à une température allant de 20°C à la température de reflux du solvant.

Office européen des brevets

**RAPPORT DE RECHERCHE EUROPEENNE**

0200605

Numero de la demande

EP 86 40 0708

## DOCUMENTS CONSIDERES COMME PERTINENTS

| Catégorie | Citation du document avec indication en cas de besoin des parties pertinentes | Revendication concernee | CLASSEMENT DE LA DEMANDE (Int Cl 4) |
|---|---|---|---|
| Y | CHEMICAL ABSTRACTS, vol. 77, no. 21, 20 novembre 1972, page 413, résumé 139686m, Columbus, Ohio, US; M D. LIPANOVA et al.: "Furohydrazides and their derivatives"; & Issled. Obl. Geterotsikl. Soedin. 1971, 28-38<br>--- | 1-4 | C 07 D 405/12<br>A 61 K 31/44 |
| Y | CHEMICAL ABSTRACTS, vol. 50, no. 13, 10 juillet 1956, colonne 9371h-9372e, Columbus, Ohio, US; H. SAIKACHI et al.: "Synthesis of furan derivatives. XIV. Condensation of 5-nitrofuranacrolein and 5-nitrofurfural with hydrazides"; & Pharm. Bull. (Japan) 3, 194-9(1955)<br>----- | 1-4 | |
| | | | **DOMAINES TECHNIQUES RECHERCHES (Int Cl.4)** |
| | | | C 07 D 405/00 |

Le present rapport de recherche a éte etabli pour toutes les revendications

| Lieu de la recherche | Date d achevement de la recherche | Examinateur |
|---|---|---|
| LA HAYE | 14-07-1986 | ALLARD M.S. |

CATEGORIE DES DOCUMENTS CITES

X particulièrement pertinent a lui seul
Y particulièrement pertinent en combinaison avec un autre document de la même categorie
A arrière plan technologique
O divulgation non-écrite
P document intercalaire
T theorie ou principe a la base de l invention
E document de brevet anterieur mais publie a la date de depôt ou apres cette date
D cité dans la demande
L cité pour d autres raisons
& membre de la même famille document correspondant

OEB Form 1503 03 82